# EUROPEAN PATENT APPLICATION

(11) **EP 3 081 173 A2**
(43) Date of publication of application: **19.10.2016**
(21) Application number: 16164753.2
(22) Date of filing: 11.04.2016
(51) Int. Cl.: A61B 17/12, A61B 17/00

(54) **DETACHMENT MECHANISM FOR VASCULAR DEVICES**

(30) Priority: 15.04.2015 US 201562147935 P; 24.03.2016 US 201615079351
(71) Applicant: Cook Medical Technologies LLC, Bloomington, IN 47404 (US)
(72) Inventor: ELGAARD, Per, 4690 Haslev (DK); TORP, Allan, 4632 Bjaeverskov (DK)
(74) Representative: Mathys & Squire LLP

(57) **Abstract**

The disclosure provides for a delivery apparatus or mechanism and method of use for delivering a medical device to the body of a patient. The apparatus may have an outer catheter independently mobile from an inner sheath (43) and an elongate member (22). The apparatus further has a gripper (36) attached to the elongate member. The gripper may be manipulated by way of a handle (80) to control the state of the medical device relative to the delivery apparatus. Through use of the handle, the apparatus may be moved from a connected state to a released state to deliver the medical device to a target site in the patient's body.

## Description

### FIELD

The present disclosure relates to medical devices. More particularly, the disclosure relates to a coaxial detachment mechanism to manipulate a medical or vascular device.

### BACKGROUND

Physicians often use implantable devices (i.e. vascular devices) to treat various conditions. Such devices may be designed to be inserted in tubular body vessels, such as arteries and veins, and delivered to a particular vessel location. Due to the small size of some body vessels and the tortuous pathways necessary to reach certain locations, there is a need for improved delivery mechanisms.

In addition, increasingly physicians are using minimally invasive procedures and percutaneous techniques to deliver medical devices to various locations within the body. In these procedures, physicians may employ a variety of outer sheaths or microcatheters to initially access a particular body location where treatment is necessary (e.g. an aneurysm). It may be desired to keep such an outer sheath in place until the physician has finished the procedure, without moving the outer sheath.

However, any additional tools that the physician inserts or delivers through the outer sheath to the intended vessel location may be dependent on what outer sheath has been chosen. Due to the pairing necessary between an outer sheath and any additional tools, the physician may desire to employ tools that are capable of being inserted into the outer sheath and manipulated independently of the outer sheath.

### BRIEF SUMMARY

The present disclosure provides generally for a delivery apparatus for delivering a medical device to a body vessel. The apparatus generally includes an outer catheter, an inner sheath, an elongate member, a handle, a gripper, and a connector piece. The inner sheath may be slidably disposed in and coaxial with the outer catheter. The inner sheath may have a proximal end and extending distally to a distal end with a lumen formed therethrough, defining a longitudinal axis.

The elongate member may be disposed in the lumen and coaxial with the inner sheath. The elongate member having a first end disposed adjacent the proximal end. The elongate member may extend distally from the proximal end to a second end disposed adjacent the distal end.

The handle may be connected to the proximal end and the first end, with one of the proximal end and the first end being slidably received by the handle. The handle may further include an actuator and a biasing member. The actuator may be attached to the one of the first end and the proximal end, and may cooperate with the biasing member such that actuating the actuator moves the one of the first end and the proximal end relative to the longitudinal axis.

The gripper may include a first member and a second member. The first member may have a first segment attached to the second end and extending distally to a second segment. The second member may be formed to cooperate with the first member such that the first and second members opposingly face each other. The second member may have a third segment attached to the second end and extending distally to a fourth segment where the first and third segments may be pivotally attached, defining a pivot area. The second and fourth segments may be detached.

The connector piece may be removably attached to the medical device and extend proximally from the device such that the first and second members cooperate to hold the connector piece therebetween. The second and fourth segments may be disposed part a distance (d1), defining a connected state. The gripper may be in the connected state when disposed partly in the inner sheath, being in contact with the connector piece. The first and second members may be formed to biasingly pivot outwardly at the pivot area relative to the longitudinal axis to release the connector piece. The second and fourth segments may be disposed apart a distance (d2). Distance (d2) may be greater than distance (d1) to release the connector piece, defining a released state. The gripper may be in the released state when disposed farther out of the inner sheath relative to the connected state, being out of contact with the connector piece.

This disclosure also provides a method for delivering a medical device to a body vessel. The method includes (1) coupling the medical device to a delivery apparatus, as discussed herein; (2) slidably moving the inner sheath and the elongate member relative to the outer catheter to position the device within the body vessel; and (3) moving one of the second end and the distal end relative to each other such that the gripper opens, the apparatus allowing independent movement between the inner sheath and the outer catheter for positioning the medical device. The method may further comprise the step of unlocking the locking member before the step of moving the gripper.

As one possible advantage, the apparatus may allow independent movement between the inner sheath and the outer catheter for positioning the medical device. Because the inner sheath is placed between the gripper and the outer catheter, the physician, or user of the device, may be able to manipulate and move the gripper without moving the outer catheter. This independent movement may create an active apparatus, which the physician can manipulate separate from the outer catheter, as opposed to a passive apparatus that limits gripper movement to the position and movability of the outer catheter.

In addition with this independent ability, a medical device may be positioned and re-coupled to the delivery apparatus without moving the outer catheter. This may allow for positioning, repositioning, and/or aborting delivery of the medical device as needed or desired by the physician.

### BRIEF DESCRIPTION OF THE DRAWINGS

Figure 1 illustrates the partial, environmental side view of a delivery apparatus and a medical device in accordance of one embodiment of the present invention.
Figures 2A-C illustrate partial, side views of one embodiment of the delivery apparatus of Figure 1.
Figure 3 illustrates a partial, side view the delivery apparatus Figure 1.
Figures 4A-B illustrate blown-up, partial, side views of the delivery apparatus of Figure 1.
Figure 5 illustrates a side view of a handle of the delivery apparatus Figure 1.
Figures 6A-B illustrate exploded views of the handle of Figure 5.
Figures 7A-B illustrate blown-up, side views of a locking member of the handle of Figure 5.
Figure 8 illustrates steps of a method of delivering the medical device with the delivery apparatus of Figure 1 in accordance of one embodiment of the present invention.

### DETAILED DESCRIPTION

The present disclosure provides for a coaxial detachment mechanism to manipulate a medical or vascular device in the body of a patient. The materials, methods, and examples disclosed herein are illustrative only and not intended to be limiting. The disclosed figures are not necessarily to scale.

All publications, patent applications, patents, and other references mentioned herein are incorporated by reference in their entirety. Unless otherwise defined, all technical and scientific terms used herein have the same meaning as commonly understood by one of ordinary skill in the art to which said disclosure pertains. In the case of conflict, the present documents and definitions will control.

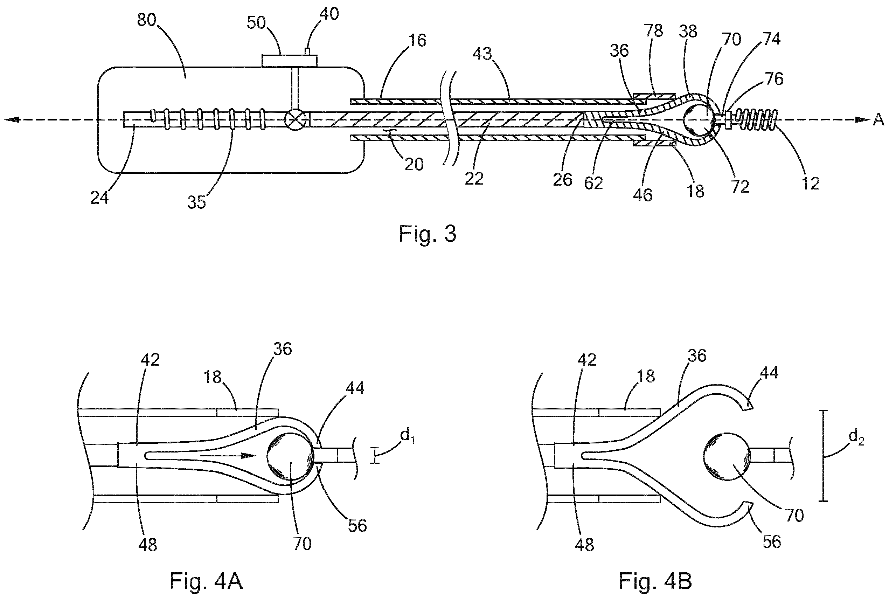

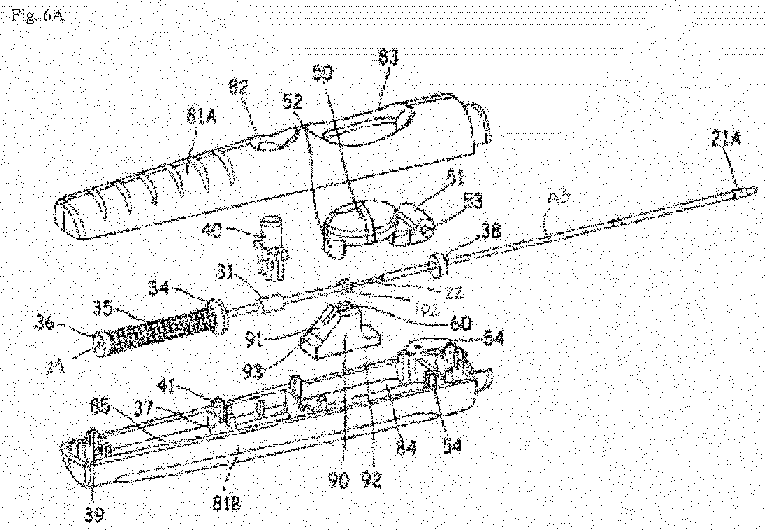

"Adjacent" referred to herein is nearby, near to, or in close proximity with. An adjacent condition may also be depicted in the figures (e.g. Figs. 3 and 6A-B).

The terms "proximal," "distal," and derivatives thereof will be understood in the frame of reference of a medical physician using a medical device; thus, proximal refers to those locations closer to the physician and distal refers to locations farther away from the physician (e.g. deeper in the patient's vasculature).

"Substantially" referred to herein means approximately, about, or close to.

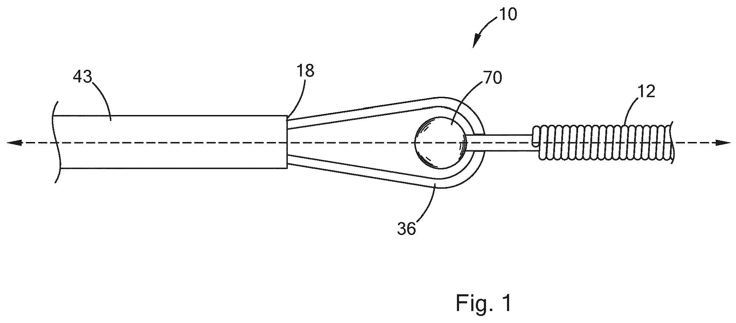

Figure 1 illustrates an environmental view of the delivery mechanism or apparatus 10. In this view, the inner sheath 43 may extend distally to a distal end 18, defining a longitudinal axis A of the apparatus 10. The gripper 36 may extend distally from the distal end 18 and attached to a connector piece 70. The connector piece 70 may be disposed on the proximal end of a medical device 12. The medical device 12 may extend distally from the rest of the delivery apparatus 10.

The medical device 12 could be any device that a physician intends to deliver to a target site within a patient (e.g. stents, filters, and the like). In Figure 1, the medical device is an occlusion coil. Such a device may be inserted into a target site in the vasculature (e.g. an aneurysm).

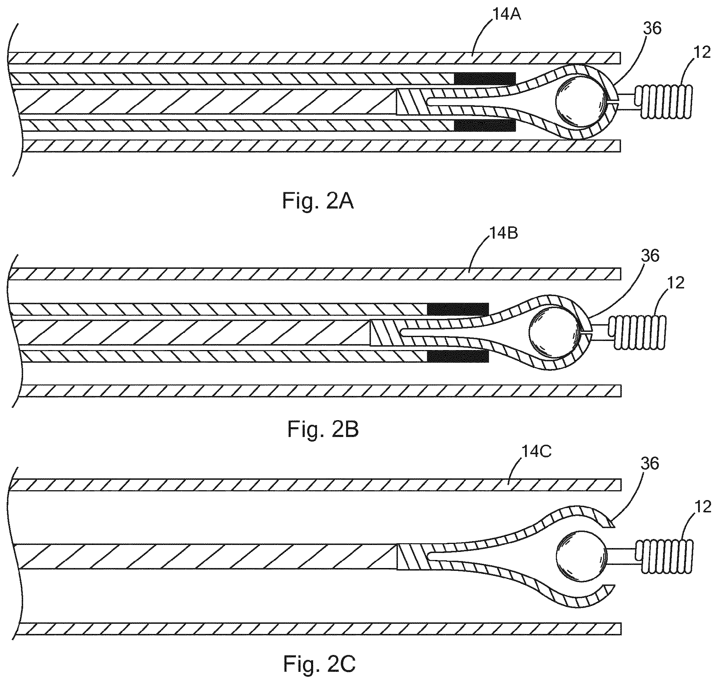

Figures 2A-B depict embodiments where an outer catheter is disposed about the inner sheath 43. In Figure 2A, this is outer catheter 14A. In Figure 2B, this is outer catheter 14B. The outer catheter may be one with any sizing known in the art. As will be apparent with the figures, outer catheter 14A may make a tight fit with the gripper 36, while outer catheter 14B may not. Regardless of the precise size of the outer catheter, the inner sheath 43 may hold the gripper 36 in position.

In one example, the outer catheter is a micro catheter. The microcatheter or outer catheter may have an inner diameter ranging from about 0.018 inches to about 0.035 inches. One skilled in the art will understand that the outer diameter of the microcatheter or outer catheter could be any size for the target site.

As may be apparent with Figure 2C, without the inner sheath, the precise sizing of the outer catheter (e.g. 14A, B, and C) may determine how well the apparatus performs. In the example illustrated here, outer catheter 14C may be oversized or too large to make a snug fit around the gripper 36. As a result, the gripper 36 may prematurely deploy the medical device 12, possibly deploying the medical device 12 before exiting the outer sheath 14C at the target delivery site. Such premature deployment may be unintended or undesired by the physician.

Figure 3 depicts a more detailed view of the delivery apparatus of Figure 1. The delivery apparatus may include an outer catheter (as in Fig. 2A), and inner sheath 43, elongate member 22, a handle 80, a gripper 36, and connector piece 70. The inner sheath 43 may be slidably disposed in and coaxial with the outer catheter (e.g. Fig. 2A, 14A). The inner sheath 43 may have a proximal end 16 extending distally to a distal end 18 and a lumen 20 formed therethrough, defining a longitudinal axis A. The inner sheath 43 may further include a marker band 78 at the distal end. The marker band 78 may be circumferentially disposed about the distal end 18 to allow the physician to visualize the distal end when disposed in the patient. The marker band 78 may be radiopaque, as known to those skilled in the art.

In addition to facilitating visualization, marker band 78 may provide a stopping or resting point for the gripper 36 (as depicted in Fig. 3). In this way, the marker band 78 may be slidably disposed about a first member 38 and a second member 46 of gripper 36. This stopping point may facilitate the connected state by allowing the physician to pull the gripper 36 against the distal end 18. In any embodiments, the marker band 78 may be disposed at the distal end 18 and may have a smaller or larger overall diameter than the rest of the inner sheath 43. This smaller or larger diameter, marker band diameter, may facilitate closing the gripper 36, allowing for wider engineering tolerances of the inner sheath 43. Of course, it may also be the case that the marker band 78 does not provide a stopping point and the gripper 36 may be completely encircled or disposed inside the inner sheath 43 in the connected state.

The elongate member 22 may be disposed in the lumen 20 coaxial with the inner sheath 43 along the longitudinal axis A. The elongate member 22 may have a first end 24 disposed adjacent the proximal end 16. The elongate member 22 may extend distally from the proximal end 16 to a second end 26 disposed adjacent the distal end 18. In one case, the elongate member 22 may be a wire. The elongate member 22 may be a seven strain cable wire. Alternatively, elongate member 22 may be a straight mandrel.

Handle 80 may be connected to the proximal end 16 and the first end 24. One of the proximal end 16 and the first end 24 may be slidably received by the handle 80, giving two alternative embodiments to the delivery apparatus 10. The handle 80 may include an actuator 50 and a biasing member 35. The actuator 50 may be attached to the one of the first end 24 and the proximal end 16 (see e.g. Fig. 3). The actuator 50 may cooperate with the biasing member 35 such that actuating the actuator 50 moves the one of the first end 24 and the proximal end 16 relative to the longitudinal axis.

Handle 80 may be activated to change the tension in the inner sheath 43 or the elongate member 22 through the biasing member 35. In the depicted embodiment in Figure 3, the biasing member 35 may be a spring. However, the biasing member 35 may be any member known in the art to change the tension of the inner sheath 43 or the elongate member 22. For example, such alternative structure may be compressed gas or a balloon. The biasing member 35 may facilitate pulling the gripper 36 against the distal end 18, partly inside the inner sheath 43. It may also facilitate pulling the gripper 36 completely inside the inner sheath 43, both possibilities as discussed herein.

The handle 80 may be rotated by the physician about the longitudinal axis during delivery or retrieval. The handle 80 may have two alternative embodiments. In a first embodiment, the actuator 50 may be attached to the first end 24 to move the first end relative to the longitudinal axis. In this embodiment, the apparatus may be configured such that the actuator may move or push the elongate member distal relative to the longitudinal axis to uncover the gripper and release the medical device.

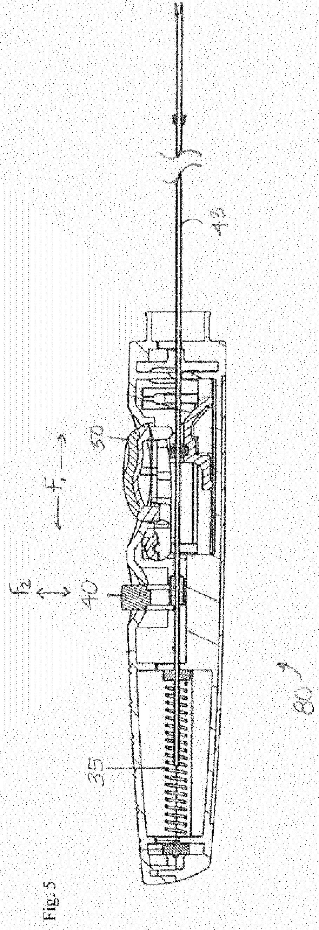

In the second embodiment, the actuator 50 may be attached to the proximal end to move the proximal end relative to the longitudinal axis. In this embodiment, the apparatus may be configured such that the actuator may move or pull the inner sheath proximal relative to the longitudinal axis to uncover the gripper and release the medical device. The handle 80 will be discussed in further detail with Figures 5-7.

The gripper 36 may include a first member 38 and a second member 46. Overall details of the apparatus are shown in Figure 3, while specific details of the gripper 36 are illustrated in Figures 4A-B. As shown in Figures 3-4B, the first member 38 may have a first segment 42 attached to the second end 26 and extend distally to a second segment 44. The second member 46 may be formed to cooperate with the first member 38 such that the first and second members opposing the face each other. The second member 46 may have a third segment 48 attached to the second end 26 and extending distally to a fourth segment 56. The first and third segments may be pivotably attached, defining a pivot area 62. The second and fourth segments may be detached.

The first member 38 and a second member 46 may be any suitable shape to connect to the connector piece 70. In this embodiment, the first member and a second member each include an arcuate portion, being arcuatly shaped. In addition, the pivot area 62 may be U-shaped. At the pivot area 62, the gripper may bend and accommodate stress stored in the bends as the gripper moves in and out of the inner sheath.

The gripper 36 may be attached to the elongate member 22 in any method and means known in the art including gluing, soldering, welding. In addition, the gripper 36 may be any material known in the art to pivot, being resilient. Such examples include Nitinol, spring steel, stainless steel, and palladium.

Nitinol is a metal alloy of nickel and titanium having a unique shape memory setting property and being biocompatible. At a transition temperature, Nitinol may undergo a phase change from Martensitic to Austenite, changing its structure. Here, the gripper 36 could be heat set or pre-set to the released state and maintained in the connected state (shown in Figures 4A-B) until released or deployed. In addition to this phase changing ability, Nitinol is also quite flexible.

The connector piece 70 may be removably attached to the medical device 12 and extend proximately from the device such that the first and second members cooperate to hold the connector piece 70 therebetween. The second and fourth segments may be disposed apart a distance (d1) apart, defining a connected state. The gripper 36 may be in the connected state when disposed partly in the inner sheath 43, disposed in contact with the connector piece 70.

The first and second members being formed to biasingly pivot outwards at the pivot area 62, relative to the longitudinal axis. The second and fourth segments may be disposed apart a distance (d2) to release the connector piece 70. The distance (d2) may be greater than the distance (d1), defining a released state. The gripper 36 may be in the released state when disposed out farther of the inner sheath 43 relative to the connected state, being disposed out of contact with the connector piece 70. The apparatus thereby allows independent movement between the inner sheath 43 and the outer catheter for positioning the medical device 12.

In Figure 3, the connector piece 70 may further include a sphere 72, a neck 74, and a joint 76. The joint 76 allows rotational freedom between the sphere 72 and the medical device 12. For example, the sphere 72 may be disposed proximal the neck 74 and being connected to the neck 74. The neck 74 may distally extend from the sphere 72 to the joint 76. The joint 76 may be connected to the medical device 12 to attach the connector piece 72 the device 12.

In this example, the neck 74 may be rotational about the longitudinal axis at the joint 76 such that the neck 74 and the sphere 72 rotate together. However, the neck 74 and the sphere 72 rotate separate, or are rotationally independent, from the medical device 12. The independent rotational movement about the longitudinal axis between the connector piece 70 and the medical device 12 allows easier movement of the apparatus through certain body portions (e.g. tortuous vasculature). This may allow the physician to rotate or induce a torque to the connector piece 70 while alleviating any stress or strain on the medical device 12 during delivery or retrieval in the connected state.

The gripper 36 being arcuate corresponds in shape to the sphere 72. This allows selective coupling between the gripper 36 and the connector piece 70, as shown in Figure 3. In addition the connector piece may be made of any material known in the art to satisfy the requirements of the connector piece. For example, the connector piece may be platinum, palladium, or stainless steel.

Figures 4A-B depicts two possible states of the apparatus: the connected state and the released state. In Figure 4A, the apparatus may be in the connected state where the sphere 70 is disposed in the gripper 36. In this state, the gripper may extend distally from the distal end 18 about 0 millimeters (mm) to about 1 millimeter. More preferably, the gripper may extend distally from the distal end 18 about 0.3 mm to about 0.5 mm in the connected state. Figure 4B depicts the released state where the sphere 70 is not connected to the gripper 36.

In the released state, the second segment 44 and the fourth segment 56 may extend distally about 1 millimeter to about 10 millimeters from the distal end 18, disposed apart the distance (d2). More preferably, the second segment 44 and the fourth segment 56 may extend distally about 2 mm to about 6 mm from the distal end 18 in the released state.

Figures 5 and 6A-B depict further details of the handle, as discussed herein. Figure 5 illustrates details of the handle 80, while Figures 6A-B illustrate exploded forms to further display the internal components thereof. It will be appreciated, however, that the handle unit can be used for the deployment and retrieval in a wide variety of locations of a patient.

In Figure 5, the handle 80 contacts inner sheath 43. The inner sheath may have sufficiently flexibility to provide the required trackability. As detailed herein, while both the inner sheath 43 and the elongate member (not shown here) are attached to the handle 80, one of the two may be slidably received in the handle 80 through a force F1 on actuator 50 in the direction of the arrow depicted (substantially parallel to the longitudinal axis). In this embodiment, actuator 50 is a first button. Such force F1 may compress or elongate the biasing member 35, shown as a spring in Figure 5. In an alternative arrangement, the actuator 50 may act by way of a force substantially perpendicular to the longitudinal axis, either in connection with the force F1 or without a need for the force F1.

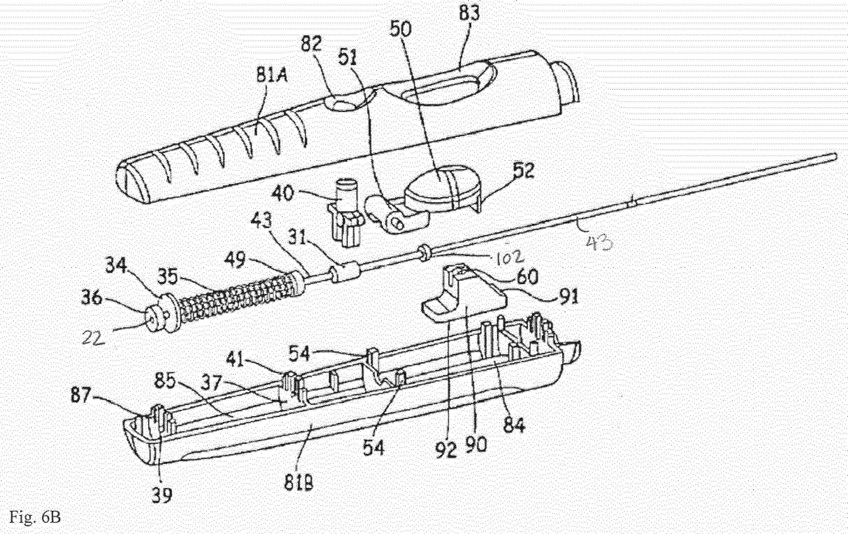

In addition, the device may comprise a locking member 40 (details of which will be discussed with Figures 6-7). The locking member 40 may have a locked state and an unlocked state. The locking member may include a trigger, at least one engagement element, and a receiver element located on the one of the inner sheath and the elongate member.

The trigger may be a second button and may be moveable in a direction toward and substantially perpendicular to the longitudinal axis to lock the one of the inner sheath and the elongate member relative to the other of the inner sheath and the elongate member by engaging the at least one engagement element with the receiver element. The trigger may also be moveable to unlock the apparatus. A force F2 on the trigger may move the locking member 40 between the locked and unlocked states, depicted by the arrow in Figure 5.

Figure 6A depicts one embodiment of the handle, where the elongate member 22 is slidably received in the handle. As depicted in Figure 6A, the handle is formed of an elongate casing formed in two halves 81A, 81 B which engage along its length. The top half 81A is provided with two holes 82, 83 which allow access to an actuator or first button 50 and a locking member 40, both described in further detail below.

The handle also houses the proximal end of the inner sheath 43, within which is mounted the flexible elongate member 22. An annular holding disc 38 is fixed to the inner sheath 43 and serves to fix the inner sheath 43 to the handle 80 in the longitudinal direction of the apparatus. The elongate member 22 is provided with a spring 35 over its proximal end, between two annular shoulder or stop discs 34, 36. The stop disc 36 is fixed to the proximal end of the elongate member 22, while the stop disc 34 is slidable on the elongate member 22.

The interior of the handle 80 is provided with first and second walls 37, 39, delimiting the ends of a spring chamber 85 in the handle 80. The two stop members 34, 36 abut against the walls 37, 39. The distal wall 37 is provided with a slot or other aperture 41 therein allowing passage of the elongate member 22.

The apparatus is also provided with a receiver or engagement element 31, described in full detail below, for use in locking the apparatus. At a location distal of the engagement element 31, there is provided a follower element 102 fixed to the elongate member 22. The follower element 102 is designed so as to fit within a recess 60 of a carriage element 90. The carriage element 90 has, in this embodiment, a substantially flat lower surface 92 which is able to slide along a track 84 within the handle, such sliding movement causing, by virtue of the coupling of the handle element 90 to the follower element 102, the inner sheath 43 to slide with the carriage 90.

The first button 50 of the handle, shown in particular in Figure 6A, is provided with a hinge 51 having two opposing pins 53 upstanding therefrom, able to engage a suitable recess (not shown) in the top portion of the handle and to rest upon supports 54 in the lower portion of the handle 80. The first button 50 is also provided with a depending follower portion 52 which cooperates with a bevelled or sloping surface 91 of the carriage element 90. The design is such that upon pressure being applied to depress the button 50, this pivots about the hinge 51, causing the depending follower member 52 to rotate, to abut and then to slide along the sloping surface 91 of the carriage 90. As this occurs, the carriage 90 is moved in a distal direction, which is to the right in the view of Figure 6A.

The proximal wall of the carriage unit 90, at the bottom end of the sloping surface 91, is provided with a recessed zone 93, in this embodiment in the form of a channel extending traverse to the direction of movement of the carriage. In operation, when the depending follower 52 reaches the end of the sloped surface 91, it suddenly falls into the recessed portion 93, providing an audible as well as a tactile jump in the movement of the button 50. This informs the physician that the button 50 has reached its fully depressed and/or extended position and, in practice, that the elongate member 22 is fully extended (not shown beyond break point 21 A).

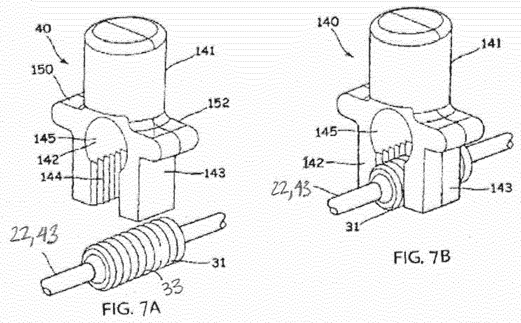

Figure 6A also shows the locking member 40, which cooperates with the engagement element 31. Here, locking member 40 may be spaced apart from actuator 50 along the handle. Specific details of the locking member 40 are described in further detail below in connection with Figures 7A-B.

In operation of the handle, when the button 50 is depressed and/or extended, the dependent element 52 slides down the sloping wall 91 and in so doing pushes the carriage 90 forwards, in a distal direction. The movement of the carriage 90, by virtue of the connection through the element 102, pushes the elongate member 22 forward. When a medical device is gripped onto the end of the elongate member 22, this is in turn disposed out of the inner sheath, eventually allowing for release of the medical device carried by the apparatus. As the carriage 90 and the first end 24 of the elongate member 22 move forwards, the coil spring 35 resiliently extends or elongates. Once the button 50 has been pushed all the way down and/or forward, the physician feels and hears the click as the depending follower 52 drops into the channel 93.

As long as the button 50 is kept depressed and the locking member 40 is not engaged (as described below), when the button 50 is released, the coil spring 35 can return to its original position, thereby pulling the follower 90 backwards, which in turn pulls backwards the elongate member 22, thereby retracting this. Such retraction is affected both for withdrawing the apparatus after deployment of the medical device into a patient and also to pull into the apparatus the medical device, which is to be retrieved from a patient. Thus, the coil spring 35 is preferably of sufficient strength to be able to pull back and collapse a medical device by the force of the spring alone.

Figure 6B show another version of a handle having similar characteristics as the handle of Figure 6A. In the embodiment of 6B, the mechanism could be said to be reversed compared to the embodiment of 6A in that it operates to pull back the inner sheath 43, rather than to push forwards the elongate member 22. In 6B, the carriage element 90 is reversed compared to the embodiment of 6A, as is the button 50, such that the carriage moves backwardly in a proximal direction upon depression and/or proximal retraction of the button 50.

The inner sheath 43 also extends to the proximal portion of the handle and is provided thereon with the engaging element 31 and with the spring retaining disc 49, which is fixed to the inner sheath 43. Another retaining disc or ring 36 is fixed to the proximal end of the inner sheath 43 and a sliding disc 34 is located between the two fixed discs 36 and 43. The disc 36 at the end of the handle fits within a chamber 85 at the end of the handle 80 so as to fix the elongate member 22 to the handle 80 such that the elongate member 22 cannot move longitudinally relative to the handle. It is the inner sheath 43 which moves relative to the handle 80.

In a similar manner to the embodiment of 6A, when the first button 50 is depressed and/or slid, leaving aside operation of the locking member 40 which will be described below, the dependent portion 52 of the first button 50 slides down the sloping surface 91 of the carriage member 90, pushing the carriage member backwards in a proximal direction, thereby pulling the inner sheath 43 in a proximal direction so as to expose the second end of the elongate member 22. A device held by the elongate member 22 can then be released or, in the case of a retrieval operation, cause the gripper (not shown here) to grab on to an implanted device so as to effect its retrieval from within the patient.

As the carriage 90 moves the inner sheath 43 backwards, this compresses the spring 35, the compression of a spring 35 being guided by the location of the proximal end of the inner sheath 43. Thus, when the button 50 is released, leaving aside any operation of the locking member 40 (described below), the spring 35 is able, by virtue of its spring force, to push the inner sheath 43 forwards, thereby to cause this to envelop again the end of the elongate member 22. When the second end of the elongate member 22 has caught a device to be retrieved, the spring 35 will cause that device, if of collapsible form, to be collapsed into the inner sheath 43 or an enveloping outer sheath, so as to capture the device within the apparatus for withdrawal from the patient.

Figures 7A-B show examples of a preferred embodiment of locking member 40. It is preferred that both the elongate member and inner sheath are kept locked longitudinally with respect to the handle 80 until the point that a physician desires to release or withdraw the medical device into or from a patient. In particular, it is preferred that the inner sheath and elongate member are not movable relative to one another from the point of assembly of the apparatus to the point at which it is to be used clinically, thus to be locked in particular during transportation and handling of the apparatus. The locking member provides such function.

The locking member 40 includes a trigger or second button 141, which is accessible from the aperture 82 in the handle. In this embodiment, the second button 141 includes first and second prong elements 142, 143 depending from the trigger or second button 141, which in this embodiment are substantially parallel to one another. The prongs 142, 143 are provided with internal surfaces having, in this embodiment, a plurality of vertically extending teeth 144, elongate teeth which extend in a direction substantially parallel to the direction of movement of the second button 141.

The engagement portion 31 provided on the elongate member 22 or inner sheath 43, in the examples of Figures 6A-B, is in the form of a grooved annular bushing, the grooves 33 of which are sized and spaced to receive respective teeth 144 of the second button 141. As can be seen in 7A-B, the second button 141 is provided with an enlarged rounded aperture 145 above the pronged elements 142, 143 and in particular above the teeth 144. The enlarged aperture 145 is larger than the maximum diameter of the engagement element 31, such that when the button 141 is depressed to such an extent that the engagement portion 31 becomes located within the enlarged aperture 145, the engagement portion 31 can slide within the aperture 145, thus allowing for sliding of the elongate member 22 or inner sheath 43.

The button 141 is also provided with shoulders 150, 152 which abut suitable surfaces in the top half of the handle 80 so as to limit the upward movement of the button 141 within the handle. The lower surfaces of the prongs 142, 144 are, in this embodiment, substantially flat and, in practice, support one or more elements (not shown) intended to bias the button upwardly. These elements thus retain the button normally in an upward-most position.

The arrangement is such that when the second button 141 is in its uppermost position, the teeth 144 engage the grooves 33 or surface of the engagement element 31, so as to lock the elongate member 22 relative to the inner sheath 43. The button 141 must be depressed, so as to locate the engagement portion 31 in the enlarged aperture 145, before the elongate member can be moved relative to the inner sheath or vice versa. Thus, a physician must first depress the locking member 40 to unlock the apparatus and only then will the depression of the first button 50 cause movement so as to deploy the medical device.

The arrangement has another advantage, namely that the arrangement of locking member 40 and engagement element 31 are not dependent upon a particular longitudinal alignment of these two components. This allows not only for manufacturing tolerances but also the use of different sizes of devices to be deployed/retrieved. It allows the device to be locked in one of a plurality of relative positions of the inner sheath and elongate member. For example, this can allow the physician to lock the elongate member 22 in its most extended position, for example, to assist in the retrieval of the medical device. Furthermore, in such a case, when the physician has determined that the device to be retrieved has connected with the elongate member 22, simple depression of the locking member 40 will cause the handle 80 to come into action. It is preferred in some embodiments that this action is swift, which can facilitate in the collapse of the device being retrieved. There are similar advantages during deployment.

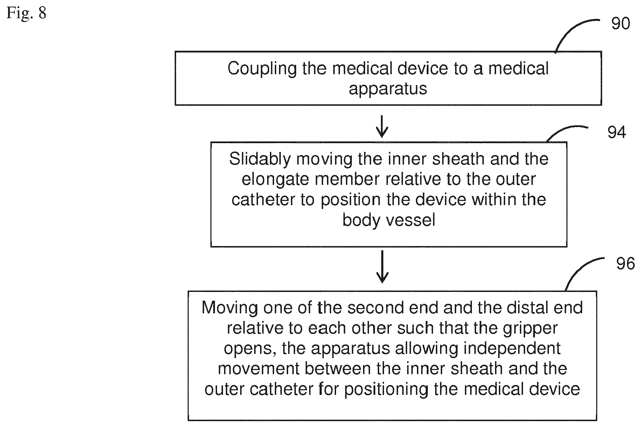

Figure 8 depict details of the steps of one delivery method for a medical device. First, in step 90, the physician may coupling the medical device to a delivery apparatus, structural details of both discussed herein. Second, in step 94, the physician may slidably moving the inner sheath and the elongate member relative to the outer catheter to position the device within the body vessel. Third, in step 96, the physician may move one of the second end and the distal end relative to each other such that the gripper opens, the apparatus allowing independent movement between the inner sheath and the outer catheter for positioning the medical device.

Due in part to the independent nature of the inner sheath from the outer catheter, the physician has the ability to release the connector piece from the gripper and, subsequently, recouple the connector piece to the gripper. As such, the physician may recouple the device to the apparatus after the step of moving one of the second end and the distal end. The physician can also release the connector piece in the patient's body, and, subsequently, recouple the device to the apparatus to abort the procedure entirely (i.e. withdrawing the medical device from the body).

In addition, the apparatus may be initially unlocked with the locking device in an unlocked position. One method of use may involve locking the locking member before the step of coupling. When the physician is ready to release the connector piece, the physician may unlock the locking member before the step of moving the gripper.

There is herein described a method for delivering a medical device to a body vessel, the method comprising: coupling the medical device to a delivery apparatus, the apparatus comprising: an outer catheter; an inner sheath being slidably disposed in and coaxial with the outer catheter, the inner sheath having a proximal end and extending distally to a distal end with a lumen formed therethrough, defining a longitudinal axis; an elongate member being disposed in the lumen and coaxial with the inner sheath, the elongate member having a first end disposed adjacent the proximal end and extending distally from the proximal end to a second end disposed adjacent the distal end; a handle being connected to the proximal end and the first end, one of the proximal end and the first end being slidably received by the handle, the handle comprising an actuator and a biasing member, the actuator being attached to the one of the first end and the proximal end and cooperating with the biasing member such that actuating the actuator moves the one of the first end and the proximal end relative to the longitudinal axis; a gripper connected to the second end and comprising a first member and a second member, the second member being cooperatively formed with the first member such that the first and second members opposingly face each other, the first member and the second member being pivotally attached, defining a pivot area; and a connector piece being removably attached to the medical device and extending proximally from the device such that the first and second members cooperate to hold the connector piece therebetween, defining a connected state, the gripper being in the connected state when disposed in contact with the connector piece, the first and second members being formed to biasingly pivot outwardly at the pivot area relative to the longitudinal axis to release the connector piece, defining a released state, the gripper being in the released state when disposed out of contact with the connector piece, the apparatus allowing independent movement between the inner sheath and the outer catheter for positioning the medical device; slidably moving the inner sheath and the elongate member relative to the outer catheter to position the device within the body vessel; and moving one of the second end and the distal end relative to each other such that the gripper opens, the apparatus allowing independent movement between the inner sheath and the outer catheter for positioning the medical device. The method may further comprise the step of recoupling the device to the apparatus after the step of moving one of the second end and the distal end.

While the present invention has been described in terms of certain preferred embodiments it will be understood that the invention is not limited to these disclosed embodiments as those skilled in the art may make various modifications without departing from the scope of the following claims.

## Claims

1. A delivery apparatus for delivering a medical device to a body vessel, the apparatus comprising:
an outer catheter;
an inner sheath being slidably disposed in and coaxial with the outer catheter, the inner sheath having a proximal end and extending distally to a distal end with a lumen formed therethrough, defining a longitudinal axis;
an elongate member being disposed in the lumen and coaxial with the inner sheath, the elongate member having a first end disposed adjacent the proximal end and extending distally from the proximal end to a second end disposed adjacent the distal end;
a handle being connected to the proximal end and the first end, one of the proximal end and the first end being slidably received by the handle, the handle comprising an actuator and a biasing member, the actuator being attached to the one of the first end and the proximal end and cooperating with the biasing member such that actuating the actuator moves the one of the first end and the proximal end relative to the longitudinal axis;
a gripper connected to the second end and comprising a first member and a second member, the second member being cooperatively formed with the first member such that the first and second members opposingly face each other, the first member and the second member being pivotally attached, defining a pivot area; and
a connector piece being removably attached to the medical device and extending proximally from the device such that the first and second members cooperate to hold the connector piece therebetween, defining a connected state, the gripper being in the connected state when disposed in contact with the connector piece, the first and second members being formed to biasingly pivot outwardly at the pivot area relative to the longitudinal axis to release the connector piece, defining a released state, the gripper being in the released state when disposed out of contact with the connector piece, the apparatus allowing independent movement between the inner sheath and the outer catheter for positioning the medical device.

2. The apparatus of claim 1 wherein the actuator is attached to the proximal end to move the proximal end relative to the longitudinal axis.

3. The apparatus of claim 1 wherein the actuator is attached to the first end to move the first end relative to the longitudinal axis.

4. The apparatus of any one of the preceding claims wherein connector piece comprises a sphere, a neck, and a joint, the sphere disposed proximal the neck and being connected to the neck, the neck distally extending from the sphere to the joint, the joint being connected to the medical device to attach the connector piece to the device.

5. The apparatus of claim 4 wherein the neck is rotational about the longitudinal axis at the joint such that the neck and the sphere rotate together, the neck and the sphere are rotationally independent from the medical device.

6. The apparatus of any one of the preceding claims wherein the biasing member is a spring.

7. The apparatus of any one of the preceding claims wherein the handle comprises a locking member to lock one of the inner sheath and the elongate member, the locking member including a trigger, at least one engagement element, and a receiver element located on the one of the inner sheath and the elongate member, the trigger moveable in a direction toward and substantially perpendicular to the longitudinal axis to lock the one of the inner sheath and the elongate member relative to the other of the inner sheath and the elongate member by engaging the at least one engagement element with the receiver element.

8. The apparatus of claim 7 wherein the trigger is moveable in the direction substantially perpendicular to the longitudinal axis to unlock the one of the inner sheath and the elongate member relative to the other of the inner sheath and the elongate member by disengaging the at least one engagement element from the receiver element.

9. The apparatus of any one of the preceding claims wherein the actuator comprises a first button activated by a first force substantially parallel to the longitudinal axis.

10. The apparatus of claim 8 wherein the trigger comprises a second button activated by a second force substantially perpendicular to the longitudinal axis.

11. The apparatus of claim 8 wherein the at least one engagement element comprises a first facing engagement element and a second facing engagement element, the first and second facing engagement elements comprising a plurality of teeth, preferably wherein the receiving element includes a plurality of grooves for receiving the teeth of the first and second facing engagement elements.

12. The apparatus of claim 1 wherein the inner sheath comprises a marker band at the distal end, the marker band being circumferentially disposed about the distal end, and preferably slidably disposed about the first and second members of the gripper.

13. The apparatus of any one of the preceding claims wherein the pivot area is U-shaped.

14. A delivery apparatus for delivering a medical device to a body vessel, the apparatus comprising:
an outer catheter;
an inner sheath being slidably disposed in and coaxial with the outer catheter, the inner sheath having a proximal end and extending distally to a distal end with a lumen formed therethrough, defining a longitudinal axis;
an elongate member being disposed in the lumen and coaxial with the inner sheath, the elongate member having a first end disposed adjacent the proximal end and extending distally from the proximal end to a second end disposed adjacent the distal end;
a handle being connected to the proximal end and the first end, one of the proximal end and the first end being slidably received by the handle, the handle comprising an actuator and a biasing member, the actuator being attached to the one of the first end and the proximal end and cooperating with the biasing member such that actuating the actuator moves the one of the first end and the proximal end relative to the longitudinal axis;
a gripper connected to the second end and comprising a first member and a second member, the first member having a first segment attached to the second end and extending distally to a second segment, the second member being formed to cooperate with the first member such that the first and second members opposingly face each other, the second member having a third segment attached to the second end and extending distally to a fourth segment, the first and third segments being pivotally attached, defining a pivot area, the second and fourth segments being detached; and
a connector piece being removably attached to the medical device and extending proximally from the device such that the first and second members cooperate to hold the connector piece therebetween, the second and fourth segments being disposed part a distance (d1), defining a connected state, the gripper being in the connected state when disposed in contact with the connector piece, the first and second members being formed to biasingly pivot outwardly at the pivot area relative to the longitudinal axis, the second and fourth segments being disposed apart a distance (d2) to release the connector piece, distance (d2) being greater than distance (d1), defining a released state, the gripper being in the released state when disposed out of contact with the connector piece, the apparatus allowing independent movement between the inner sheath and the outer catheter for positioning the medical device.

15. The apparatus of claim 14 wherein the second and the fourth segments of the gripper distally extend about 1 millimeter to about 10 millimeters from the distal end in the released state, and preferably about 2 millimeters to about 6 millimeters.
